# DEMANDE DE BREVET EUROPEEN

(11) **EP 0 621 254 A2**
(43) Date de publication de la demande: **26.10.1994**
(21) Numéro de dépôt: 94400629.5
(22) Date de dépôt: 24.03.1994
(51) Int. Cl.: C07C 51/353, C07C 61/40, C07C 67/333, C07C 69/757, C07C 253/30, C07C 255/39

(54) **Procédé d'isomérisation de doubles liaisons de pyréthrionoides**

(30) Priorité: 25.03.1993 FR 9303432
(71) Demandeur: ROUSSEL UCLAF, F-93230 Romainville (FR)
(72) Inventeur: Cosquer, Philippe, F-93200 Saint Denis (FR); Demassey, Jacques, F-77144 Montevrain (FR); Demoute, Jean-Pierre, F-93360 Neuilly Plaisance (FR)
(74) Mandataire: Vieillefosse, Jean-Claude

(57) **Abrégé**

L'invention a pour objet un procédé d'isomérisation de doubles liaisons caractérisé en ce que l'on soumet un composé de formule (I) : 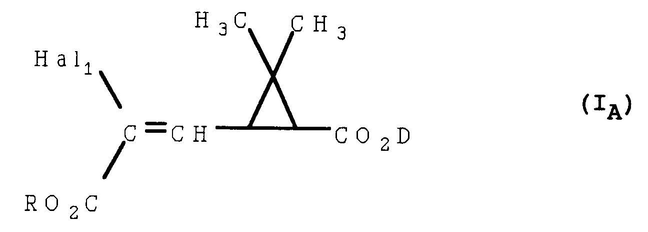
dans laquelle la géométrie de la double liaison est E ou un mélange E + Z, Hal représente un atome d'halogène, A représente un groupement électroattracteur, Hal précédant A dans la classification de Cahn Ingold et Prelog, Z₁ et Z₂ différents l'un de l'autre, représentent un atome d'hydrogène ou un radical organique, Z₁ précédant Z₂ dans la classification de Cahn Ingold et Prelog, à l'action du brome en présence de lumière pour obtenir le composé de formule (I) dans laquelle la géométrie de la double liaison est Z.

Le procédé de l'invention trouve son utilisation dans la préparation de produits biologiquement actifs par exemple des pesticides.

## Description

La présente invention concerne un nouveau procédé d'isomérisation de doubles liaisons.

L'invention a pour objet un procédé d'isomérisation de doubles liaisons caractérisé en ce que l'on soumet un composé de formule (I) : 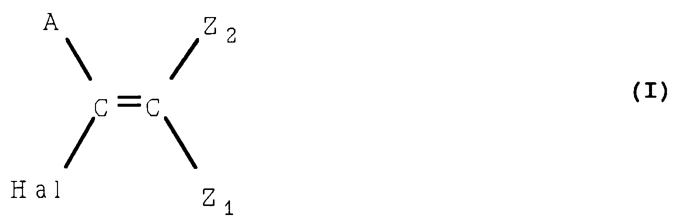
dans laquelle la géométrie de la double liaison est E ou un mélange E + Z, Hal représente un atome d'halogène, A représente un groupement électroattracteur, Hal précédant A dans la classification de Cahn Ingold et Prelog, Z₁ et Z₂ différents l'un de l'autre, représentent un atome d'hydrogène ou un radical organique, Z₁ précédant Z₂ dans la classification de Cahn Ingold et Prelog, à l'action du brome en présence de lumière pour obtenir le composé de formule (I) dans laquelle la géométrie de la double liaison est Z.

Hal représente de préférence un atome de fluor, de chlore ou de brome,

Par groupement électro attracteur, on entend par exemple un groupement CO₂R, R représentant un radical alkyle renfermant jusqu'à 8 atomes de carbone éventuellement substitué par un ou plusieurs groupes fonctionnels, ou bien un groupement C≡N, ou bien un groupement alkyle renfermant jusqu'à 8 atomes de carbone substitué par un ou plusieurs atomes d'halogène, ou bien un groupement aryle éventuellement substitué par un ou plusieurs groupes fonctionnels.

A représente de préférence un groupement CO₂R tel que défini ci-dessus ou un groupement alkyle substitué tel que défini ci-dessus.

Lorsque Z₁ ou Z₂ représente un groupement organique, il s'agit par exemple d'un groupement aryle éventuellement substitué par un ou plusieurs groupements fonctionnels, ou d'un groupement 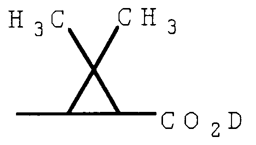
dans lequel D représente un atome d'hydrogène, un radical alkyle ou le reste d'un alcool utilisé en série pyréthrinoïde.

Dans la définition des divers substituants A, Z₁ et Z₂, l'atome d'halogène est un atome de fluor, de chlore ou de brome ou encore un atome d'iode ; par groupement fonctionnel, on entend de préférence un atome d'halogène, un groupement OH ou SH, un groupement ORa ou SRa dans lequel Ra représente un radical alkyle renfermant de 1 à 8 atomes de carbone, un groupement NO₂ ou 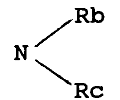
dans lequel Rb et Rc identiques ou différents représentent un atome d'hydrogène ou un radical alkyle renfermant de 1 à 8 atomes de carbone, un groupement C≡N, SO₃H ou PO₄H₂ ou un groupement COalc₁, SO₂alc₂ ou SO₃alc₃ dans lesquels alc₁, alc₂ et alc₃ représentent des radicaux alkyle renfermant de 1 à 18 atomes de carbone.

Le procédé de l'invention est très général et peut s'appliquer à tout composé organique renfermant une double liaison pouvant présenter une géométrie E, ainsi qu'un mélange de géométrie E+Z.

En appliquant le procédé de l'invention, on obtient des composés de géométrie Z au départ de composés de géométrie E ou de géométrie E+Z.

Le procédé de l'invention trouve tout son intérêt pour préparer par exemple des composés biologiquement actifs lorsque les composés de géométrie Z sont plus actifs que les composés de géométrie E, ou encore pour préparer des produits intermédiaires de synthèse des produits finis. C'est ainsi par exemple que le procédé de l'invention permet de préparer les composés décrits dans les brevets européens 50534, 402246, 178826, 299694 ou 10874.

L'invention a notamment pour objet un procédé caractérisé en ce que le brome est utilisé en quantité catalytique.

L'invention a également pour objet un procédé caractérisé en ce que l'on utilise le brome en quantité stoechiométrique et en ce que l'on soumet le produit obtenu à l'action d'un agent de réduction pour obtenir le composé de formule (I) dans laquelle la géométrie de la double liaison est Z.

La réaction où le brome est utilisé en quantité catalytique ainsi que la réaction où le brome est ajouté en quantité stoechiométrique, sont mises en oeuvre au sein d'un solvant qui peut être un solvant halogéné, comme le chlorure de méthylène, le chloroforme, le tétrachlorure de carbone, le dichloroéthane ou un mélange de ces divers solvants, un éther comme le tétrahydrofuranne, le diméthoxyéthane, le méthylterbutyl éther ou le dioxanne, un solvant aromatique tel que le benzène, le toluène, le xylène ou un solvant saturé correspondant comme le cyclohexane, un alcool comme le méthanol ou l'éthanol, ou encore l'acétate d'éthyle, le diméthylformamide ou le diméthylsulfoxyde.

Ces réactions sont mises en oeuvre par exemple sous irradiation d'une lampe à lumière blanche de 100 watts à 400 watts et de préférence de 200 à 300 watts.

Dans le cas où le brome est ajouté en quantité stoechiométrique, la réduction des produits dibromés obtenus est effectuée dans les mêmes solvants que ci-dessus à une température comprise entre -20 et +40°C, à l'aide par exemple de zinc dans l'acide acétique.

Parmi les procédés préférés de l'invention, on peut citer un procédé dans lequel le composé de formule (I) concerné est un composé dans lequel Z₂ représente un atome d'hydrogène et notamment un procédé caractérisé en ce que le composé de formule (I) concerné est le composé de formule (I_{A}) : 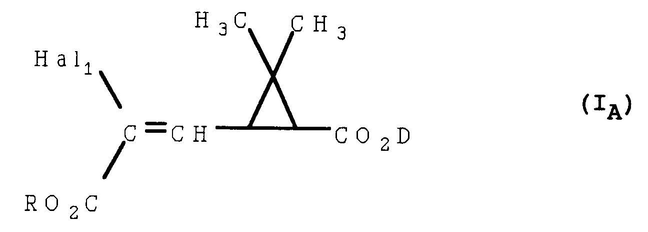
dans laquelle Hal₁ représente un atome d'halogène, D représente un atome d'hydrogène, un radical alkyle inférieur ou le reste d'un alcool utilisé en série pyréthrinoïde et R représente un radical alkyle éventuellement substitué par un ou plusieurs atomes d'halogène.

Hal₁ représente de préférence un atome de fluor, de chlore ou de brome.

Lorsque D représente un radical alkyle, il s'agit de préférence d'un radical méthyle, éthyle, n-propyle, isopropyle, n-butyle, isobutyle ou terbutyle.

Lorsque D représente le reste d'un alcool utilisé en série pyréthrinoïde, D représente de préférence :
a) soit un radical benzyle éventuellement substitué sur les sommets aromatiques par un ou plusieurs radicaux choisis dans le groupe constitué par les radicaux alkyles comportant de 1 à 4 atomes de carbone, le radical méthylènedioxy et les atomes d'halogène,
b) soit un groupement 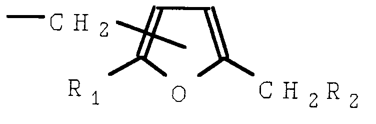 dans lequel le substituant R₁ représente un atome d'hydrogène ou un radical méthyle et le substituant R₂ un aryle monocyclique,
c) soit un groupement 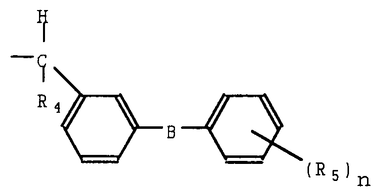 (R, S ou RS)
   dans lequel B représente un atome d'oxygène ou de soufre ou un groupement 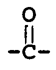 ou -CH₂-, R₄ représente un atome d'hydrogène, un atome de chlore, de brome ou d'iode, un radical -C≡N, un radical méthyle, un radical -CONH₂, un radical -CSNH₂, R₅ représente un atome d'halogène ou un radical méthyle et n représente un nombre égal à 0, 1 ou 2,
d) soit un groupement 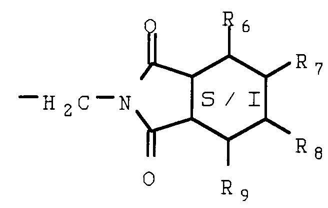 dans lequel les substituants R₆, R₇, R₈ et R₉ représentent un atome d'hydrogène, un atome de chlore ou un radical méthyle et dans lequel S/I symbolise un cycle aromatique
e) soit un groupement (succimido ou maléimido) méthylène,
f) soit un groupement 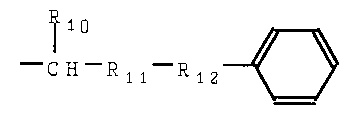 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 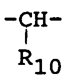 peut se trouver à l'une quelconque des positions disponibles, R₁₂ étant lié à R₁₁ par l'atome de carbone compris entre l'atome de soufre et un atome d'azote,
g) soit un groupement 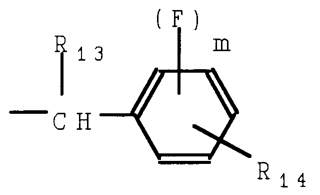 dans lequel R₁₃ représente un atome d'hydrogène ou un radical -CN, R₁₄ représente un atome d'hydrogène, un atome d'halogène, un radical C≡N, un radical alkyle, alkoxy, alkoxyalkyle, alkylthio, CO-alkyle ou CO₂-alkyle renfermant jusqu'à 6 atomes de carbone, dont le ou les radicaux alkyle sont éventuellement substitués par un ou plusieurs atomes d'halogène et m représente un nombre égal à 1, 2, 3 ou 4,
h) soit un groupement 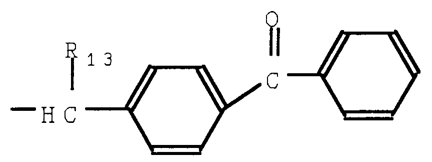 dans lequel R₁₃ est défini comme ci-dessus,
i) soit un groupement 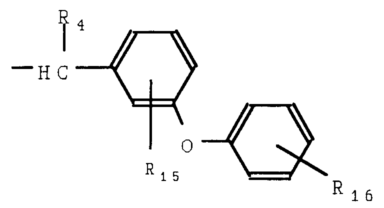 dans lequel R₄ est défini comme ci-dessus, R₁₅ représente un atome de fluor, de chlore ou de brome et R₁₆ représente un atome d'hydrogène, de fluor, de chlore ou de brome,
j) soit un groupement 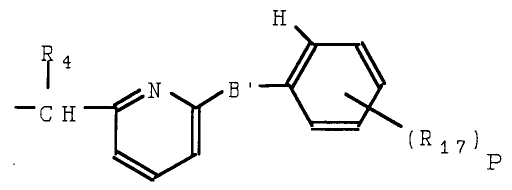 dans lequel R₄ est défini comme ci-dessus, chacun des R₁₇ représente indépendamment un groupement alkyle renfermant de 1 à 4 atomes de carbone, alcoxy renfermant de 1 à 4 atomes de carbone, alkylthio renfermant de 1 à 4 atomes de carbone, alkylsulfonyl renfermant de 1 à 4 atomes de carbone, trifluorométhyle, 3,4-méthylènedioxy, chloro, fluoro ou bromo, p représente un nombre égal à 0, 1 ou 2 et B' représente un atome d'oxygène ou un atome de soufre,
k) soit un groupement 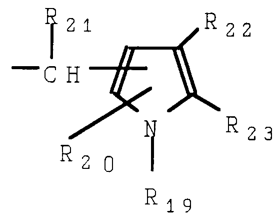 dans laquelle R₂₁ représente un atome d'hydrogène, un groupement -C≡N, -CF₃ ou un radical alkyle renfermant de 1 à 3 atomes de carbone, R₂₀, R₂₂ et R₂₃ identiques ou différents les uns des autres, représentent un atome d'hydrogène, un atome d'halogène, un radical alkyle renfermant jusqu'à 18 atomes de carbone, un radical aryle renfermant jusqu'à 14 atomes de carbone, un radical aralkyle renfermant jusqu'à 18 atomes de carbone, un radical cyano, un radical -CF₃, un radical -CO₂-alkyle renfermant jusqu'à 8 atomes de carbone, un radical NO₂, un radical alcoxy renfermant jusqu'à 8 atomes de carbone, un radical 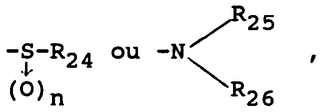 n étant égal à 0, 1 ou 2 et les radicaux R₂₄, R₂₅ et R₂₆ représentant un radical alkyle renfermant de 1 à 8 atomes de carbone, les radicaux R₂₂ et R₂₃ pouvant former un homocycle carboné saturé ou insaturé renfermant jusqu'à 8 atomes de carbone et R₁₉ représente
   - ou bien un radical 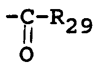 dans lequel R₂₉ peut avoir les valeurs indiquées précédemment pour R₂₂ et R₂₃, à l'exception d'halogène, cyano, NO₂, 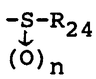 dans lequel n est égal à 1 ou 2 et 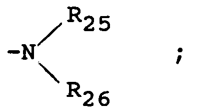
   - ou bien un radical 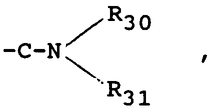 dans lequel R₃₀ et R₃₁, identiques ou différents l'un de l'autre, représentent un atome d'hydrogène, un radical alkyle renfermant de 1 à 18 atomes de carbone, un radical aryle renfermant jusqu'à 14 atomes de carbone, un radical aralkyle renfermant jusqu'à 18 atomes de carbone, un radical CF₃, un radical CO₂-alkyle renfermant jusqu'à 8 atomes de carbone ou un radical alcoxy renfermant jusqu'à 8 atomes de carbone,
l) soit un groupement 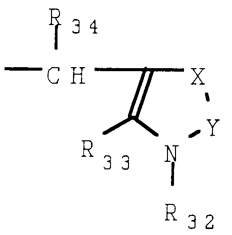 dans laquelle X représente un atome de soufre ou d'oxygène, Y représente un groupe >C=O, >C=S ou >CH₂, R₃₂ représente un atome d'hydrogène, un radical alkyle, linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, R₃₃ représente un atome d'hydrogène, un radical alkyle linéaire, ramifié ou cyclique, saturé ou insaturé, renfermant jusqu'à 8 atomes de carbone, éventuellement substitué par un ou plusieurs halogènes, ou R₃₃ représente un radical aryle renfermant jusqu'à 14 atomes de carbone, un radical -CF₃, -NO₂, -C≡N, un atome d'halogène, un radical alcoxyle renfermant jusqu'à 8 atomes de carbone ou un radical -CO₂-alkyle renfermant jusqu'à 8 atomes de carbone, R₃₄ représente un atome d'hydrogène, un radical alkyle renfermant de 1 à 3 atomes de carbone,
m) soit un groupement 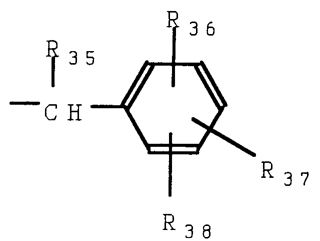 dans lequel R₃₅ représente un atome d'hydrogène, un radical alkyle renfermant de 1 à 3 atomes de carbone, un radical -C≡N ou CF₃, R₃₆ et R₃₈, identiques ou différents l'un de l'autre, représentent un atome d'hydrogène, un radical alkyle renfermant de 1 à 3 atomes de carbone éventuellement substitué par un ou plusieurs atomes d'halogène et R₃₇ représente un radical phényle éventuellement substitué par un ou plusieurs radicaux alkyle renfermant de 1 à 3 atomes de carbone ou par un ou plusieurs atomes d'halogène.

Lorsque D représente un radical benzyle substitué par un ou plusieurs radicaux alkyle, il s'agit de préférence des radicaux méthyle, éthyle, propyle ou isopropyle.

Lorsque D représente un radical benzyle substitué par un ou plusieurs atomes d'halogène, il s'agit de préférence d'atomes de chlore, de brome ou de fluor.

Lorsque R₂ représente un radical aryle, il s'agit de préférence du radical phényle et le groupement est alors de préférence le groupement 5-benzyl 3-furylméthyle.

B représente notamment un atome d'oxygène et le groupement correspondant est alors de préférence 3-phénoxybenzyle, α-cyano 3-phénoxybenzyle, α-éthynyl 3-phénoxybenzyle, 1-(3-phénoxyphényl) éthyle ou α-thioamido 3-phénoxybenzyle. B représente encore notamment un groupe -CO- et le groupement correspondant est alors de préférence 3-benzoylbenzyle.

Lorsque R₁₄ ou R₁₇ représente un radical alkyle, il s'agit de préférence d'un radical méthyle, éthyle, propyle, linéaire ou ramifié ou butyle linéaire ou ramifié.

Lorsque R₁₄ ou R₁₇ représente un radical alcoxy, il s'agit d'un radical méthoxy, éthoxy, propoxy linéaire ou ramifié ou butoxy linéaire ou ramifié.

Lorsque R₁₄ ₒᵤ R₁₇ représente un radical alkylthio, il s'agit d'un radical méthylthio, éthylthio, propylthio linéaire ou ramifié ou butylthio linéaire ou ramifié.

Lorsque R₁₇ représente un radical alkylsulfonyle, il s'agit d'un radical correspondant à l'un quelconque des radicaux alkylthio ci-dessus.

Lorsque R₂₁ représente un radical alkyle, il s'agit d'un radical méthyle, éthyle, propyle ou isopropyle et de préférence du radical méthyle.

Lorsqu'un ou plusieurs radicaux R_{2O}, R₂₂ et R₂₃ ou contenus dans les définitions de R₁₉, R₂₀, R₂₂ et R₂₃ représentent un atome d'halogène, il s'agit de préférence d'un atome de fluor, de chlore ou de brome.

Lorsqu'un ou plusieurs radicaux R₂₀, R₂₂, R₂₃ et R₂₉ ou contenus dans les définitions de R₁₉, R₂₀, R₂₂, R₂₃ et R₂₉ représentent un radical alkyle, il s'agit de préférence d'un radical méthyle, éthyle, propyle, isopropyle, butyle, isobutyle ou n-pentyle.

Lorsqu'un ou plusieurs radicaux R₂₀, R₂₂, R₂₃ et R₂₉ ou contenus dans les définitions de R₁₉, R₂₀, R₂₂, R₂₃ et R₂₉ représentent un radical aryle, il s'agit de préférence du radical phényle et celui-ci peut être substitué notamment par un radical alkyle ou alcoxy renfermant de 1 à 8 atomes de carbone, ou par un radical nitro, trifluorométhyle, hydroxy, halogéno ou amino.

Lorsqu'un ou plusieurs radicaux R₂₀, R₂₂, R₂₃ et R₂₉ ou contenus dans les définitions de R₁₉, R₂₀, R₂₂, R₂₃ et R₂₉ représentent un radical aralkyle, il s'agit de préférence du radical benzyle.

Lorsqu'un ou plusieurs radicaux R₁₄, R₂₀, R₂₂, R₂₃ et R₂₉ ou contenus dans les définitions de R₁₉, R₂₀, R₂₂, R₂₃ et R₂₉ représentent un radical -CO-alkyle,un radical -CO₂-alkyle ou un radical alcoxy, on entend de préférence par alkyle, un radical méthyle, éthyle, propyle ou isopropyle et par alcoxy, un radical méthoxy, éthoxy, propoxy ou isopropoxy.

Lorsque R₃₂ ou R₃₃ représente un radical alkyle linéaire, ramifié ou cyclique, saturé ou insaturé, il s'agit de préférence d'un radical méthyle, éthyle, n-propyle, isopropyle, cyclopropyle, cyclopropylméthyle, butyle, isobutyle, tertbutyle, cyclobutyle, n-pentyle, cyclopentyle, n-hexyle ou cyclohexyle.

Lorsque ces radicaux sont substitués par un ou plusieurs atomes d'halogène, on entend par halogène, le fluor, le chlore, le brome ou l'iode.

Lorsque R₃₂ ou R₃₃ représente un radical aryle, il s'agit de préférence du radical phényle.

Lorsque R₃₃ représente un atome d'halogène, il s'agit de préférence d'un atome de fluor, de chlore ou de brome.

Lorsque R₃₃ représente un radical alcoxy, il s'agit de préférence d'un radical méthoxy, éthoxy, propoxy ou isopropoxy.

Lorsque R₃₃ représente un radical CO₂-alkyle, par alcoyle on entend de préférence un radical méthyle, éthyle, propyle linéaire ou ramifié ou butyle linéaire ou ramifié.

Lorsque R₃₄ représente un radical alkyle, il s'agit de préférence d'un radical méthyle.

Lorsque R₃₆ et/ou R₃₈ représentent un radical alkyle, il s'agit de préférence d'un radical méthyle ou éthyle.

Lorsque le radical alkyle est substitué par un ou plusieurs atomes d'halogène, on entend de préférence par halogène, le fluor, le chlore ou le brome.

Lorsque R est substitué par un ou plusieurs atomes d'halogène, il peut s'agir par exemple du radical CH₂CCl₃, CH₂CF₃, CH₂CH₂CCl₃, CH₂CH₂CF₃, CH₂CHCl₂, CH₂CHF₂, CHF₂, CH₂CH₂Cl, CH₂CH₂F,

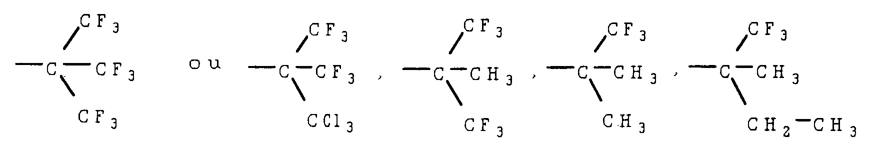

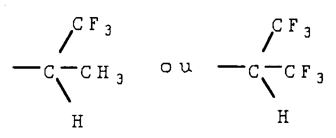

L'invention a plus particulièrement pour objet un procédé de préparation des composés de formule (I_{A}) telle que définie précédemment dans laquelle Hal₁ est un atome de fluor.

L'invention a également tout spécialement pour objet un procédé de préparation, caractérisé en ce que le composé concerné est le composé de formule (I_{B}) : 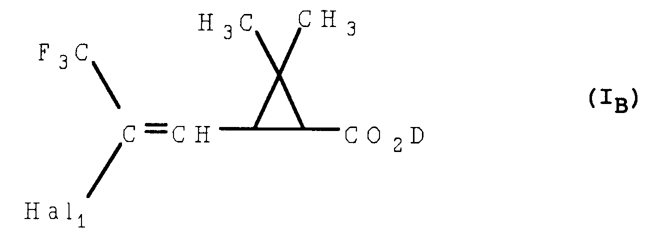
dans laquelle Hal₁ et D conservent la même signification que précédemment.

L'invention a tout spécialement pour objet un procédé de préparation des composés de formule : 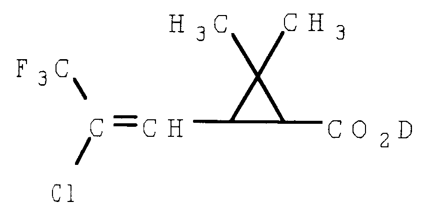
ainsi qu'un procédé de préparation des composés de formule :

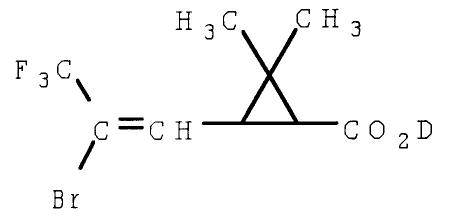

L'invention a tout particulièrement pour objet un procédé de préparation caractérisé en ce que D est un atome d'hydrogène, un radical alkyle renfermant jusqu'à 4 atomes de carbone ou un radical α-cyano 3-phénoxy benzyle.

Lorsqu'il s'agit d'appliquer le procédé de l'invention à des dérivés pyréthrinoïdes, la copule cyclopropanique peut être de configuration cis ou trans ou un mélange cis et trans.

Les exemples suivants illustrent l'invention sans toutefois la limiter.

### EXEMPLE 1: acide 1R (1alpha,3béta (Z) 2,2-diméthyl 3-(2-fluoro 3-oxo 3-méthoxy propényl) cyclopropane carboxylique

On introduit 10,8 g d'acide 1R (1alpha,3béta (E) 2,2-diméthyl (2-fluoro 3-oxo 3-méthoxypropényl) cyclopropanecarboxylique, dans 150 cm³ de tétrachlorure de carbone. On irradie avec de la lumière blanche et introduit 6,4 cm³ d'une solution de brome dans le tétrachlorure de carbone 0,39 mole/l. On maintient sous agitation pendant 15 minutes avant de concentrer sous pression réduite. Le produit brut obtenu est recristallisé dans 43 cm³ d'hexane et 21,6 cm³ d'éther isopropylique. On essore et obtient 7,39 g de cristaux blancs. F = 67°C.

| RMN CDCl₃ : | |
|---|---|
| 2CH₃gem | 1,26 (s) 1,36 (s) |
| H₁ | 1,75 (d,J = 5) |
| H₃ | 2,45 (dd,J = 5 et 10) |
| COOCH₃ | 3,83 (s) |
| =CH | 5,87 (dd,J = 10 et 31). |

### EXEMPLE 2: Acide (1R (1alpha,3béta Z)) 2,2-diméthyl 3-(2-fluoro 3-oxo 3-méthoxypropényl) cyclopropane carboxylique

### STADE A : Acide (1R(1alpha,3béta (R-S) et (1R(1alpha,3béta (S-R)) 2,2-diméthyl 3-(1,2-dibromo 3-oxo 3-méthoxy 2-fluoropropényl) cyclopropane carboxylique

On introduit 4,2 g d'acide (1R (1alpha,3béta E) 3-(2-fluoro 3-oxo 3-méthoxypropényl) cyclopropane carboxylique dans 60 cm³ de tétrachlorure de carbone.

On refroidit à 10°C ± 2°C et introduit, sous irradiation avec de la lumière blanche, une solution renfermant 1 cm³ de brome dans 6,5 cm³ de tétrachlorure de carbone. On maintient sous agitation pendant 1 heure à 10°C et concentre sous pression réduite. On obtient 9,11 g de produit brut que l'on chromatographie sur silice en éluant avec un mélange hexane, acétate d'éthyle, acide acétique (80-20-1).

On obtient le produit recherché avec un rendement de 93 %.

| RMN CDCl₃ : | |
|---|---|
| CH₃gem | 1,33 - 1,34 - 1,36 |
| H₁ | 1,65 (d,J = 5,5) cycle |
| | 1,93 (d,J = 5,5) CIS |
| H₃ | 2,21 (dd,dédoublé) |
| CO₂Me | 3,93 (s) |
| >CH-X | 4,35 (dd,dédoublé) couplé avec le fluor |

### STADE B : Acide (1R (1alpha,3béta Z)) 2,2-diméthyl 3-(2-fluoro 3-oxo 3-méthoxypropényl) cyclopropane carboxylique

On refroidit à 10°C, une solution renfermant 2,05 g d'acide préparé au stade A, 42 cm³ de chloroforme et 21 cm³ d'acide acétique. On introduit 2,10 g de zinc. On maintient l'agitation pendant 1 heure à 10°C et filtre. On rince avec du chloroforme et de l'eau. On acidifie le filtrat avec de l'acide chlorhydrique 2N jusqu'à pH 1. On décante la phase organique, sèche sur sulfate de magnésium et concentre sous pression réduite. On reprend le produit avec du toluène et concentre sous presssion réduite.

On isole 0,95 g d'acide recherché soit un rendement brut de 79 %.

### EXEMPLE 3: acide 1R (1alpha,3alpha (Z) 3-(2-chloro 3,3,3-trifluoro prop-1-èn-1-yl) 2,2-diméthylcyclopropane carboxylique

On introduit 6 g d'acide 3-(2-chloro 3,3,3-trifluoro prop-1-èn-1-yl) 2,2-diméthyl cyclopropane carboxylique sous forme d'un mélange (ΔE : 40 %/Δz : 57,5 %), dans 75 cm³ de tétrachlorure de carbone. On refroidit la solution entre 0°C et +5°C et on introduit 3,2 cm³ d'une solution (0,4 M) de brome dans le tétrachlorure de carbone. On irradie à l'aide d'une lampe de 250 watts la solution obtenue, maintenue sous agitation pendant 1 heure entre 0°C et +5°C. On distille sous pression réduite à 40 ∼ 45°C. On obtient 6,28 g de produit auquel on ajoute 15 cm³ d'hexane. On chauffe au reflux jusqu'à dissolution, puis refroidit à -20°C ± 2°C, maintient l'agitation pendant 1 heure. On essore et rince à l'hexane à -20°C. On sèche sous pression réduite à 20/25°C. On obtient 5,2 g de produit dans lequel la géométrie de la double liaison est Z.

| RMN CDCl₃ ppm : | |
|---|---|
| CH₃gem | 1,32 (s) |
| H₁ | 1,99 (d) |
| H₃ | 2,24 (m) |
| CHΔZ | 6,85 (dd) |

En opérant comme précédemment, à partir de mélanges E ou E + Z appropriés, on a obtenu les produits suivants :

### EXEMPLE 4: acide 1R (1alpha,3alpha) (Z) 2,2-diméthyl 3-(2-fluoro 3-oxo 3-éthoxy propényl) cyclopropane carboxylique

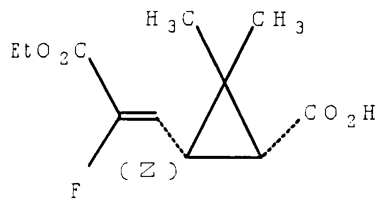

### EXEMPLE 5: acide 1R (1alpha,3alpha) (Z) 2,2-diméthyl 3-(2-fluoro 3-oxo 3-tert butyloxy propényl) cyclopropane carboxylique

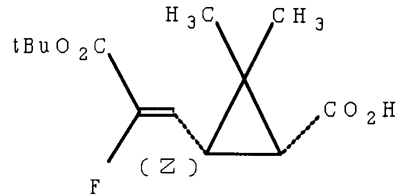

### EXEMPLE 6: 1R (1alpha,3alpha) (Z) 2,2-diméthyl 3-(2-fluoro 3-oxo 3-méthoxy propényl) cyclopropane carboxylate de alpha cyano 3-phénoxybenzyle

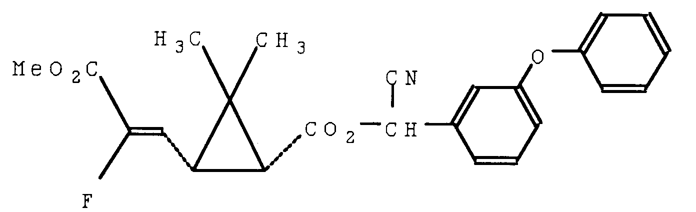

### EXEMPLE 7: 1R (1alpha,3alpha) (Z) 2,2-diméthyl 3-(2-fluoro 3-oxo 3-éthoxy propényl) cyclopropane carboxylate de alpha cyano 3-phénoxybenzyle

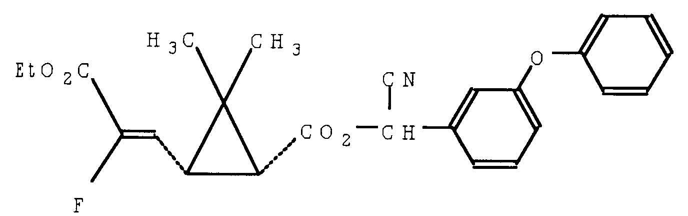

### EXEMPLE 9: 1R (1alpha,3alpha) (Z) 2,2-diméthyl 3-(2-fluoro 3-oxo 3-tert butoxy propényl) cyclopropane carboxylate de alpha cyano 3-phénoxybenzyle

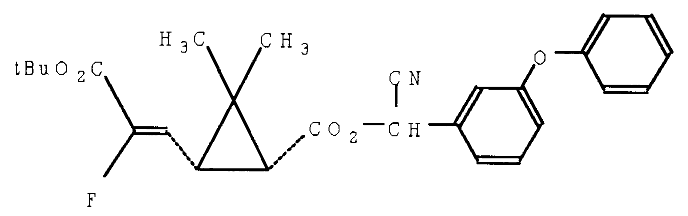

## Revendications

**1)** Procédé d'isomérisation de doubles liaisons caractérisé en ce que l'on soumet un composé de formule (I) : 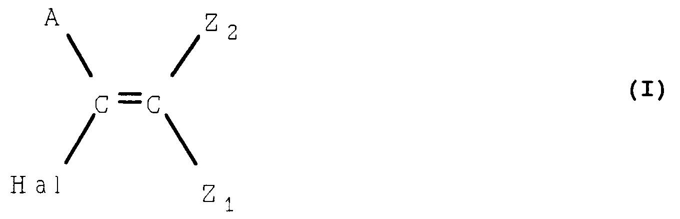 dans laquelle la géométrie de la double liaison est E ou un mélange E + Z, Hal représente un atome d'halogène, A représente un groupement électroattracteur, Hal précédant A dans la classification de Cahn Ingold et Prelog, Z₁ et Z₂ différents l'un de l'autre, représentent un atome d'hydrogène ou un radical organique, Z₁ précédant Z₂ dans la classification de Cahn Ingold et Prelog, à l'action du brome en présence de lumière pour obtenir le composé de formule (I) dans laquelle la géométrie de la double liaison est Z.

**2)** Procédé selon la revendication 1, caractérisé en ce que le brome est utilisé en quantité catalytique.

**3)** Procédé selon la revendication 1, caractérisé en ce que l'on utilise le brome en quantité stoechiométrique et en ce que l'on soumet le produit obtenu à l'action d'un agent de réduction pour obtenir le composé de formule (I) dans laquelle la géométrie de la double liaison est Z.

**4)** Procédé selon l'une quelconque des revendications 1 à 3 dans lequel Z₂ représente un atome d'hydrogène.

**5)** Procédé selon l'une quelconque des revendications 1 à 4 caractérisé en ce que le composé de formule (I) concerné est le composé de formule (I_{A}) : 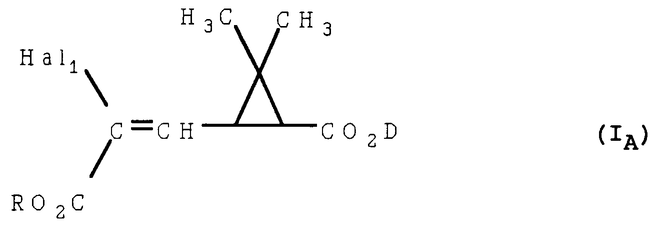 dans laquelle la copule cyclopropanique peut être de configuration cis ou trans ou d'un mélange cis et trans et dans laquelle Hal₁ représente un atome d'halogène, D représente un atome d'hydrogène, un radical alkyle inférieur ou le reste d'un alcool utilisé en série pyréthrinoïde et R représente un radical alkyle éventuellement substitué par un ou plusieurs atomes d'halogène.

**6)** Procédé selon la revendication 5 dans laquelle Hal₁ est un atome de fluor.

**7)** Procédé selon l'une quelconque des revendications 1 à 4, caractérisé en ce que le composé concerné est le composé de formule (I_{B}) : 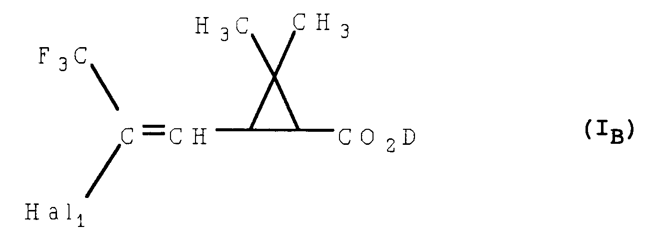 dans laquelle la copule cyclopropanique peut être de configuration cis ou trans ou d'un mélange cis et trans et dans laquelle Hal₁ et D conservent la même signification que dans la revendication 5.

**8)** Procédé selon la revendication 7, caractérisé en ce que Hal₁ est un atome de chlore.

**9)** Procédé selon la revendication 7, caractérisé en ce que Hal₁ est un atome de brome.

**10)** Procédé selon l'une quelconque des revendications 5 à 9, caractérisé en ce que D est un atome d'hydrogène, un radical alkyle renfermant jusqu'à 4 atomes de carbone ou un radical αcyano 3-phénoxy benzyle.
